# EUROPEAN PATENT APPLICATION

(11) **EP 0 820 773 A1**
(43) Date of publication of application: **28.01.1998**
(21) Application number: 96938224.1
(22) Date of filing: 15.11.1996
(51) Int. Cl.: A61K 48/00, C12N 15/54, C12N 15/85, C12N 15/90, C12N 9/12, C12N 5/10, C12N 7/01, C07K 14/47

(54) **TREATMENT OF DIABETES WITH A GLUCOKINASE GENE**

(30) Priority: 16.11.1995 US 559368
(71) Applicant: Universidad Autonoma Barcelona, 08193 Bellaterra (ES)
(72) Inventor: BOSCH TUBERT, Fátima, 08193 Bellaterra (ES); VALERA ABRIL, Alfons, 08193 Bellaterra (ES)
(74) Representative: Elzaburu Marquez, Alberto
(86) International application number: ES9600216
(87) International publication number: WO9717994

(57) **Abstract**

A process of treating a diabetic patient. The process includes the step of administering to the patient a DNA segment which includes a glucokinase gene and a promoter sequence. In the DNA segment, the promoter sequence is operably linked to the glucokinase gene and is effective for the expression of a therapeutically effective amount of glucokinase in the diabetic patient.

## Description

### Background of the invention

Hyperglycemia is found in both insulin-dependent diabetes mellitus and non-insulin-dependent diabetes mellitus. Hyperglycemia develops when sugar entering the blood stream after a meal is not adequately removed by the liver and peripheral insulin-sensitive tissues such as muscle and fat (Bennett, P.H., In Diabetes Mellitus, edited by Ellenberg, et al., 408-414 (3d ed., Medical Examination Publishing Co. 1983)).

The liver takes up excess sugar from the circulation and stores it as glycogen. When glucose enters the liver cells, it is phosphorylated by the enzyme glucokinase to glucose-6-PO₄. Glucose-6-PO₄ forms the substrate for glycogen synthesis and glucose utilisation. The storage of glucose in the liver, however, is perceived to account for only a fraction of the glucose removed from the blood stream (Flier, et al., N. Engl. J. Med. 327:707-713 (1992)).

Most of the glucose removed from the bloodstream is stored in muscle and fat. The disposal of sugar into muscle and fat is regulated by insulin produced from the pancreas (Mueckler, M., J. Diab. Comp. 7:130-141 (1993)). Insulin supplementation will increase the uptake of sugar by peripheral tissues leading to an alleviation of the symptoms of diabetes mellitus. Therefore, the classical view of the disease is principally one of insulin insufficiency with emphasis on the muscle and fat as major tissues for the maintenance of glucose homeostasis (Holman, et al., Diabetic Medicine 2:45-53 (1985)).

### Summary of the Invention

An aspect of the invention features a process of treating a diabetic patient. The process includes the step of administering to the patient a DNA segment containing a glucokinase gene and a promoter sequence, in which the promoter sequence is operably linked to the glucokinase gene and is effective for the expression of a therapeutically effective amount of glucokinase in the diabetic patient. It was unexpected that the expression of glucokinase in a diabetic patient would have helped normalise the patient's glucose level in the absence or independent of the insulin level. The term "therapeutically effective amount" means an amount of the expressed glucokinase which is sufficient to effect glucose uptake into cells, tissue, or organ of the patient, and can be determined without undue experimentation.

The glucokinase coding sequence of the DNA segment can be the same or substantially the same as the coding sequence of the endogenous glucokinase coding sequence as long as it encodes a functional glucokinase proteins. Indeed, the DNA segment can also be the same or substantially the same as the glucokinase gene of a non-human species as long as it encodes a functional glucokinase protein. The transcription of the glucokinase gene in the DNA segment is preferably under the control of a promoter sequence different from the promoter sequence controlling the transcription of the endogenous coding sequence, e.g., a promoter sequence which remains activated or induced during diabetic conditions in the patient, such as elevated levels of glucose, glucagon, triglyceride, or free fatty acids. Examples of such promoter sequences include the phosphoenolpyruvate carboxykinase ("PEPCK") promoter and the myosin light chain ("MLC") promoters, e.g., MLC1, MLC2, and MLC1/3 promoters.

In one embodiment, the DNA segment is introduced to the diabetic patient in cells, wherein the cells are treated *in vitro* to incorporate therein the DNA segment and, as a result, the cells express *in vivo* in the diabetic patient a therapeutically effective amount of glucokinase. The DNA segment can be introduced into the cells by a viral vector, e.g., a retroviral vector. The cells can also express a glucose transporter, e.g., GLUT-1, GLUT-2, GLUT-3, GLUT-4, or GLUT-5. Examples of cells expressing GLUT-2 are liver cells, kidney cells, intestine cells, and cells transfected to express GLUT-2. Examples of cells expressing GLUT-4 are muscle cells, fat cells, and cells transfected to express GLUT-4. The cells can also be administered to the diabetic patient in an intact mass as a neo-organ. E.g., see Mueckler, J. Diab. Comp. 7:130-141 (1993).

In another embodiment, the DNA segment is directly introduced to the diabetic patient, e.g., not contained within a cell. The DNA segment can be introduced in a vector. Examples of suitable vectors include viral vectors (e.g., retroviral vectors, adenoviral vectors, adenoassociated viral vectors, sindbis viral vectors, and herpes viral vectors), plasmids, cosmids, and yeast artificial chromosomes. The DNA segment can also be introduced as infectious particles, e.g., DNA-ligand conjugates, calcium phosphate precipitates, and liposomes.

Also contemplated within the scope of this invention are the DNA segments descried herein, as well as vectors and cells containing such a DNA segment.

Other features and advantages of the present invention will be apparent from the brief description of the drawings, the detailed description of the invention, and from the claims.

### Brief Description of the Drawings

The figures are first briefly described:
Fig. 1 is a diagrammatic representation of the construction of a DNA vector carrying a glucokinase gene for expression into liver cells.
Fig. 2 is a diagrammatic representation of the construction of a DNA vector carrying a glucokinase gene for expression into muscle cells.

### Detailed Description of the Invention

The methods of making and using DNA segments to practice the therapeutic process of this invention are well within the ability of a person or ordinary skill in the art. Unless defined otherwise, all technical and scientific terms used herein have the same meaning as commonly understood by one of ordinary skill in the art to which this invention belongs. Also, all publications cited herein are incorporated by reference.

The therapeutic process of the invention allows for the overexpression (e.g., at a higher level than pretreatment) of glucokinase in a diabetic patient. The over-expression of glucokinase in the diabetic patient results in the uptake of glucose into the cells expressing the glucokinase DNA segment.

What is meant by "DNA segment" herein is any exogenous DNA construct which includes a sequence encoding for a functional glucokinase, and the glucokinase is expressed by the cells into which the DNA segment is introduced. The DNA segment can be introduced into both the somatic and germ cells or only into some of the somatic cells of the patient, or cells expressing the DNA segment can be introduced *ex vivo* into the patient. The DNA segment, therefore, may or may not be an integral part of the patient's chromosome, and if the DNA segment is integrated into a chromosome, it may or may not be located at the same site as its corresponding endogenous gene sequence.

The DNA segment used to practice the therapeutic process includes a glucokinase gene or its complementary DNA ("cDNA"), whose expression is driven by a promoter which is expressed during diabetic conditions. Examples of suitable promoters include the general constitutively active promoters/enhancers, e.g., the $-actin promoter (Kawamoto, et al., Mol. Cell Biol. 8:267-272 (1988); Morishita, et al., Biochem. Biophys. Acta 1090:216-222 (1991)), cytomegalo virus ("CMV") and SV40 promoters, (Okayama, et al., Mol. Cell Biol. 3:280-289 (1983); and Boshart, et al., Cell 41:521-530 (1985)), or retroviral long terminal repeat sequences (LTR) (Crystal, RG, Science 270:404-410 (1995)), and strong tissue specific constitutive promoters, e.g., the muscle-specific myosin light chain promoters (Lee, et al., J. Biol. Chem. 267:15875-15885 (1992); Greishammer, et al., Cell 69:79-93 (1992)) and the liver specific albumin promoter (Heckel, et al., Cell 62:447-456 (1991).

The promoter is comprised of a cis-acting DNA sequence which is capable of directing the transcription of a gene in the appropriate environment, tissue, context, and in response to physiological regulators, e.g., hormones, glucose, and intermediary biochemical metabolizers. The expression of a transgene (e.g., a glucokinase gene or its cDNA sequence) can be regulated. Examples of inducible promoters include the glucagon inducible phosphoenolpyruvate carboxykinase promoter (Valera, et al., Proc. Natl. Acad. Sci. USA, 91:9151-9154 (1994), the divalent metal inducible metallothionein promoter (Karin, et al., Proc. Natl. Acad. Sci. USA 80:4040-4044 (1993)), and the tetracycline inducible promoter (Grossen, et al., Science 268 (1995)).

The genomic DNA sequence and cDNA sequences of the glucokinase gene for various species have been reported. Examples include the glucokinase DNA sequence in human (Koranyi, et al., Diabetes 41:807-811 (1992); Nishi, et al., Diabetologia 35:743-747 (1992); Stoffel, et al., Proc. Natl. Acad. Sci. USA 89:7698-7702 (1992); Tanizawa, et al., Proc. Natl. Acad. Sci. USA 88:7294-7297 (1991); and Tanizawa, et al., Mol. Endocrinol. 6:1070-1081 (1992)) and in rat (Andreone, et al., J. Biol. Chem. 264:363-369 (1989); Magnuson, et al., J. Biol. Chem. 264:15936-15942 (1989); Hayer, et al., Biochem. J. 270:261-263 (1990); Iynedjian, et al., J. Biol. Chem. 262:6032-6038 (1987)).

Examples of cells targeted for overexpression of glucokinase include hepatocytes from the liver (Peng, et al., Proc. Natl. Acad. Sci. USA 85:8146 (1988); Wolff, et al., Proc. Natl. Acad. Sci. USA 84:3344 (1987); and Wilson, et al., Proc. Natl. Acad. Sci. USA 85:3014 (1988)), myoblast and myocytes from the muscle (Salminer, et al., Human Gene Therapy 2:15-26 (1991)), stem cells of the bone marrow (Bakx, et al., Human Gene Therapy 2:301-306 (1991); Williams, et al., Nature 310:476-480 (1984); Lim, et al., Proc. Natl. Acad. Sci. USA 86:8892-8896 (1989); Demarguoy, et al., Human Gene Therapy 3:3-10 (1992); and Wieder, Human Gene Therapy 2:323-326 (1991)), fibroblasts from the skin (Wolff, et al., Proc. Natl. Acad. Sci. USA 87:2877-2881 (1990); Morgan, et al., Science 237:1476-1479 (1987); and Green, Scientific American 265:96-102 (1991)), neuronal cells from the brain (Price, et al., Proc. Natl. Acad. Sci. USA 84:156-160 (1987); and Renfranz, et al., Cell 66:713-729 (1991)), and endothelial cells (Nabel, et al., Science 244:1342-1344 (1989)).

To practice the therapeutic process of this invention, one can use vectors (both viral and non-viral DNAs, e.g., plasmids, cosmids, and yeast artificial chromosomes) and other gene delivery systems available for either the *in vitro* expression into cells utilized in *ex vivo* implantation or direct *in vivo* delivery of a glucokinase gene into the cells or tissues of a patient. Detailed guidance is provided below:

### Viral Vectors for the Delivery of a Glucokinase Gene

Viral vectors can be used for the delivery of a glucokinase gene. Examples of viral vectors include recombinant retroviral vectors, recombinant adenoviral vectors, recombinant adeno-associated viral vectors, sindbis viral vectors, and recombinant herpes viral vectors.

### (a) Recombinant Retrovirus Vectors

The genome of recombinant retroviral vector is comprised of long terminal repeat ("LTR") sequences at both ends which serve as a viral promoter/enhancer and a transcription initiation site and a Psi site which serves as a virion packaging signal and a selectable marker gene (e.g., a neomycin resistance gene). An example of such vector is pZIP-NeoSV (Cepko, et al., Cell 53:103-1062 (1984)). The glucokinase gene can be cloned into a suitable cloning site in the retroviral genome. Expression is under the transcriptional control of the retroviral LTR. The vector will drive the constitutive expression of glucokinase in the appropriate cell type. The level of expression is dictated by both the promoter strength of the LTR. Tissue selectivity is determined by both the origin of the viral genome (e.g., sarcoma virus, leukemia virus, or mammary tumor virus) and the cell line used to package the virus.

Specific modifications in the LTR sequence to improve the level of expression of the cloned gene have been described (Hilberg, et al., Proc. Natl. Acad. Sci. USA 84:5232-5236 (1987); Holland, et al, Proc. Natl. Acad. Sci. USA 84:8662-8666 (1987); and Valerio, et al., Gene 84:419-427 (1989)). The glucokinase gene can also be cloned into the vector linked to an internal promoter as an expression cassette (Crystal, R.G., Science 270:404-410 (1995)). The use of an internal promoter has also been shown to confer an additional level of control on gene expression (Lai, et al., Proc. Natl. Acad. Sci. USA 86:10006-10010 (1989); and Scharfmann, et al., Proc. Natl. Acad. Sci. USA 88:4626-4630 (1991)). Examples of internal promoter are strong constitutive promoters, e.g., the $-Actin promoter (Kawamoto, et al., Mol. Cell Biol. 8:267-272 (1988); Morishita, et al., Biochem. Biophys. Acta 1090:216-222 (1991); Lai, et al., Proc. Natl. Acad. Sci. USA 86:10006-10010 (1989)), the H-2k promoter (Schuh, et al., Nature 345:757-760 (1990); Jat, et al., Proc. Natl. Acad. Sci. USA 88:5096-5100 (1991)), and the CMV and SV40 promoters (Miller, et al., Biotechniques 7:980-990 (1989)). The promoter can also be an inducible-regulatable promoter, e.g., the mouse metallothionein promoter (Karin, et al., Proc. Natl. Acad. Sci. USA 80:4040-4044 (1983)), the tetracycline inducible promoter (Gossen, et al., Science 1766-1769 (1995); Efrat, et al., Proc. Natl. Acad. Sci. USA 92:3576-3580 (1995)), a tissue specific promoter/enhancer, e.g., the liver specific mouse albumin promoter (Heckel, et al., Cell 62:447-456 (1991)) and PEPCK promoter (Valera, et al., FASEB J. 8:440-447 (1994)), the muscle specific myosin light chain promoters (Lee, et al., J. Biol. Chem. 267:15875-15885 (1992); Shen, et al., Mol. Cell Biol. 11:1676-1685 (1991); Lee, et al., Mol. Cell Biol. 14:1220-1229 (1994); Grieshammer, et al., Cell 69:79-93 (1992); and Donoghue, et al., Gene Dev. 2:1779-1790 (1988)), the alpha-myosin heavy chain promoter (Molkentin, et al., J. Biol. Chem. 268:2602-2609 (1993)), or the fat specific adipocyte P2 promoter (Graves, et al., Genes Dev. 5:428-437 (1991); Ross, et al., Proc. Natl. Acad. Sci. USA 87:9590-9594 (1990)). Examples of such retroviral vectors incorporating an internal promoter include vLPGKSN (Valera, et al., Euro. J. Biochem. 222:533-539 (1994)) and mLBSN (Ferrari, et al., Human Gene Therapy 6:733-742 (1995)).

Recombinant retroviruses capable of transducing the glucokinase gene are produced by transfecting the recombinant retroviral genome(s) into a suitable (helper-virus free) amphotropic packaging cell line. Examples of virus packaging cell lines include PA317 and Psi CRIP (Cornetta, et al., Human Gene Therapy 2:5-14 (1991), Miller, et al., Mol. Cell Biol. 6:2895-2902 (1986); Cone, et al., Proc. Natl. Acad. Sci. USA 81: 6349-6353 (1984)). The transfected virus packaging cell line will package and produce recombinant retroviruses, shedding them into the tissue culture media. The retroviruses are harvested and recovered from the culture media by centrifugation (Compere, et al., Mol. Cell Biol. 9:6-14 (1989)). The viruses are resuspended in a suitable buffer, e.g., 10 mM HEPES, and stored at -70EC or in liquid nitrogen.

Retrovirus vectors can offer a wide host range and tissue tropism with the appropriate choice of internal promoter and virus packaging cell line. Selective targeting is achieved by modification of the envelope protein produced by the packaging cell line. For example, through the generation of a chimeric envelope protein with a single chain variable fragment from a monoclonal antibody recognizing the human low density lipoprotein receptor, it was possible to efficiently target infection of the cells expressing the receptor (Somia, et al., Proc. Natl. Acad. Sci. USA 92:7570-7574 (1995)).

### (b) Recombinant Adenovirus Vectors

Adenovirus can be used as a vector for transducing a glucokinase expression cassette. A number of adenovirus vectors have been developed for the transduction of genes into cells (Berkner, et al., BioTechniques 6:616-629(1988)). Constitutive high level expression of the transduced gene products has been achieved. These vectors have the inherent advantage over the retroviral vectors in not requiring replicating cells for infection, making them suitable vectors for somatic gene therapy (Mulligan, R.C., Science 260:926-932 (1993)).

Replication defective adenoviruses lacking the E1 region of the genome have been developed which will accommodate an insertion of 7.5 kilobases of foreign DNA (Crystal, R.G., Science 270:404-410 (1995); Logan, et al., Proc. Natl. Acad. Sci. USA 81:3655-3659 (1994); Freidman, et al., Mol. Cell Biol. 6:3791-3797 (1986); Levrero, et al, Gene 101:195-202 (1991); and Imler, et al., Human Gene Therapy 6:71-721 (1995)). These replication defective recombinant adenoviruses can be propagated by transfecting the genome into cells engineered to express the E1 genes (Jones, et al., Cell 16:683 (1979); and Berkner, et al., BioTechniques, 6:616-629 (1988)). This system allows the production of adenovirus particles at high titer (e.g., up to 10¹³/ml), which greatly enhances infection efficiency by enabling a higher multiplicity of infection (Crystal, R.G., Science 270:404-410 (1995)).

Strategies for generating adenoviral recombinants have been described (Berkner, et al., BioTechniques 6:616-629 (1988)). An example is the use of the plasmid pMLP6 (Logan, et al., Proc. Natl. Acad. Sci. USA 81:3655-3659 (1984)) which carries the Adenovirus 5 genome with the E1 region deleted. Digestion with the restriction endonucleases *Bgl*II and *Rsa*I will produce a linearized plasmid that retains only the left most 194 bp of the Adenovirus 5 genome. An expression cassette containing a regulated tissue specific promoter region, e.g., the PEPCK promoter (Valera, et al., FASEB 8:440-447 (1994)), linked to a DNA fragment encoding glucokinase with compatible 3' and 5' ends (modified by appropriate linker ligations and then subjected to appropriate restriction endonuclease digestion as described in Maniatis, et al., Molecular Cloning-A Laboratory Manual (Cold Spring Harbor Laboratory, Cold Spring Harbor, 1989)) can be cloned into the Adenovirus 5 plasmid. The entire recombinant Adenovirus genome is then generated by mixing the linearized Adenovirus 5-PEPCK-Glucokinase plasmid with a subgenomic fragment of Adenovirus DNA representing the 3.85-100 map units (prepared by digesting the In340 viral genome with *Cla*I or *Xba*I) (N.E. Biolabs, Beverly, MA) (Berkner, et al., BioTechniques 6:616-629 (1988)). The DNAs are then transfected into human kidney 293 cells (Graham, et al., J. Gen. Virol. 36:59-72 (1977)) as described in Berkner, et al., Nuc. Acid. Res. 11:6003-6020 (1983). Intermolecular recombination across appropriate segments of the plasmid and the subgenomic fragment of adenoviral DNA will result in the production of replication defective recombinant adenoviral genomes carrying the PEPCK-Glucokinase chimeric gene. The recombinant genomes will emerge from the 293 cell lines as packaged viral particles shedded into the medium. Modifications of this design have resulted in high level expression vectors (Berkner, et al., BioTechniques 6:616-629 (1988)) by incorporating regions of the major late promoter and the tripartite leader elements (Berkner, et al., Nuc. Acid Res. 11:6003-6020 (1983); and Logan, et al., Proc. Natl. Acad. Sci. USA 81:3655-3659 (1984)) in the vector construction.

The use of recombinant adenoviruses for delivery of genes into cells of the airway in humans and animals have been described (Reviewed in Crystal, R.G., Science 270:404-410 (1995); Katkin, et al, Human Gene Therapy 6:985-995 (1995)). The feasibility for transducing genes associated with glycogen metabolism, using adenovirus-mediated transfer in primary rat hepatocytes and myoblast in culture, has also been described (Baque, et al., Biochem. J. 304 (Pt 3):1009-1014 (1994); Gomez-Foix, et al., J. Biol. Chem. 267:25129-25134 (1992)).

### (c) Recombinant Adeno-Associated Viruses

Adeno-associated virus ("AAV") can also be used as a vector for transducing a glucokinase expression cassette. AAV offers the advantage that it has not been implicated in the etiology of any disease, and its site specific integration on human chromosome 19 has been shown not to interfere with host gene expression or promote gene rearrangements (Kotin, et al., Proc. Natl. Acad. Sci. USA 87:2211-2215 (1990); Samulski, et al., Euro. Mol. Biol. Org. J. 10:3941-3950 (1991)). As with adenoviruses, AAV is capable of infecting post-mitotic cells, thereby, making it a suitable vector for delivery of genes to somatic cells.

The AAV genome contains two genes, *rep* and *cap*, and inverted terminal repeats (ITR) sequences (Hermonat, et al., J. Virol. 51:329-339 (1984)). Recombinant AAV vectors are constructed by replacing the *rep* gene, the *cap* gene, or both with a glucokinase gene expression cassette (Hermonat, et al., Proc. Natl. Acad. Sci. USA 81:6466-6470 (1984)). The sole sequence needed for AAV vector integration is the terminal 145 base ITR (Muzyczka, Curr. Top. Microbiol. Immunol. 158(97):97-129 (1992)). Such vectors are available in the plasmid form (Tratschin, et al., Mol. Cell Biol. 5:3251-3260 (1985); Lebkowski, et al., Mol. Cell Biol. 8:3988-3996 (1988); and McLaughlin, et al., J. Virol. 62:1963-1973 (1988)).

The recombinant AAV genomes can be packaged into AAV particles by co-transfection of the vector plasmid and a second packaging plasmid carrying the *rep* and *cap* genes into an adenovirus-infected cell. Such particles have been shown to efficiently transduce heterologous genes into a number of mammalian cell lines (Tratschin, et al., Mol. Cell Biol. 5:3251-3260 (1985); Lebkowski, et al., Mol. Cell Biol. 8:3988-3996 (1988); McLaughlin, et al., J. Virol. 62:1963-1973 (1988); Flotte, et al., Am. J. Respir. Cell. Mol. Biol. 7:349-356 (1992)).

In addition to using an expression cassette, high levels of expression of genes linked directly to the endogenous AAV p40 promoter has been demonstrated (Wondisford, et al., Mol. Endocrinol. 2:32-39 (1988)).

### (d) Herpes Virus Vectors

Herpes virus ("HSV") vectors constitute a unique system for the delivery of genes into cells of neuronal lineage (Anderson, et al., Cell Mol. Neurobiol. 13:503-515 (1993). HSV-derived vectors infect post-mitotic neurons and produce an established latent infection in some cell types making it a suitable system for somatic gene therapy (Leib, et al., BioEssays 15:547-554 (1993)).

Strategies for the generation of HSV vectors and recombinant viruses suitable for the transduction of the glucokinase gene have been described (Leib, et al., Bio-Essays 15:547-554 (1993)). The general method extensively used for mutagenizing endogenous viral genes (Post, et al., Cell 25:227-232 (1981)) can be applied for the introduction of exogenous glucokinase genes into the HSV genome.

The glucokinase expression cassette is cloned into a plasmid containing a portion of the HSV genome such that at least 300 bp flank the 5'- and 3' ends of the cassette (Breakfield, et al., New Biol. 3:203-218 (1991); and Efstathiou, et al., Brit. Med. Bull. 51:45-55 (1995)). The plasmid is transfected into permissive cells in culture along with the full length HSV DNA (Geller., et al., Proc. Natl. Acad. Sci. USA 87:8950-8954 (1990)). Homologous recombination and DNA replication will result in the generation of recombinant HSV genomes that are packaged into novel virus particles by the cell. Through several round of plaque purification, a recombinant virus carrying the glucokinase expression cassette can be identified for large scale production.

Defective HSV vectors have been successfully used to transfer exogenous genes into neurons *in vitro* and *in vivo* (Geller, et al., Proc. Natl. Acad. Sci. USA 87:1149-1153 (1990); Geller, et al., Science 241:1667-1669 (1988); and Efstathiou, et al., Brit. Med. Bull. 51:45-55 (1995)). A variety of constitutive promoters have been used including the lytic cycle HSV promoters, the Rous sarcoma virus LTR, the human cytomegalo virus (HCMV) IE promoters, and the neurofilament and PGK promoters for transient expression. Long term expression have been obtained using the Moloney murine leukemia virus LTR, HSV LAT promoter, HCMV IE promoter fused to the LAT promoter elements, and the neuron specific enolase promoter. These vectors have been reported to be useful for transduction of genes into cells of non-neuronal origin, e.g., mouse hepatocytes (Efstathiou, et al., Brit. Med. Bull. 51:45-55 (1995); Miyanohara, et al., New Biol. 4:238-246 (1992)).

### (e) Sindbis Virus Vectors Expressing Glucokinase:

Sindbis virus-based vectors are intended as self-amplifying systems to enhance expression of exogenous genes in mammalian cells (Herweijer, et al., Human Gene Therapy 6:1161-1167 (1995)). In this system, the subgenomic sequence coding for the structural proteins are replaced by the expression cassette of the transgene, e.g., glucokinase (Huang, et al., Virus Genes 3:85-91 (1989); and Bredenbeek, et al., J. Virol. 67:6439-6446 (1993)). The recombinant sindbis virus is generated by placing the entire genome under the control of the bacteriophage T7 or SP6 promoters to enable transcription of the (+) strand RNA in vitro (Herweijer, et al., Human Gene Therapy 6:1161-1167 (1995). The resultant RNA genomes are then used to transfect target cells (Xiong, et al., Science 243:1188-1191 (1989)). Infectious viruses are produced by infecting with a helper virus (Bredenbeek, et al., J. Virol. 67:6439-6446 (1993)). Modifications of this design using the Rous sarcoma virus LTR to direct the transcription of the non-structural genes have been described (Herweijer, et al., Human Gene Therapy 6:1161-1167 (1995)).

To generate a recombinant Sindbis virus vector, the luciferase gene cloned into the unique *Xba*I site in the vector pSin-Lux (Herweijer, et al., Human Gene Therapy 6:1161-1167 (1995)) is replaced by the glucokinase cDNA or an expression cassette encoding glucokinase upon appropriate restriction endonuclease modifications. See Sambrook, et al., Molecular Cloning - A Laboratory Manual (Cold Spring Harbor Laboratory, 1989).

Sindbis virus vectors have been successfully used to transduce foreign genes into 3T3 cells (mouse fibroblast), 293 cells (human kidney cell line), HepG2 cells (human hepatoma cell line), and primary rat myoblasts (Herweijer, et al., Human Gene Therapy 6:1161-1167 (1995)).

### In Vivo Delivery of a Glucokinase Gene by Viral Infection

Viral vectors can be used to deliver the glucokinase coding sequence into the cells, tissues, and organ of diabetic patients by *in vivo* infection. In *in vivo* infection, the recombinant viral vector is administered to the organism in order to result in a general systemic infection or organ/tissue specific infection of the patient. For example, intravenous injection of recombinant retrovirus or aerosol administration of recombinant adenoviral vectors results in infection of the epithelial lining of the respiratory tract (Rosenfield, et al., Science 252:431-434 (1991); and Hsu, et al., J. Infectious Dis. 166:769-775 (1992)); stereotaxic inoculation of recombinant herpes simplex viruses have resulted in infection of select regions of the brain (Fink, et al., Human Gene Therapy 3:11-19 (1992)); and infection of mitotically active (regenerating) liver by direct injection or administration through the portal vein of recombinant retroviruses has resulted in persistent expression of the infecting genome (Kalenko, et al., Human Gene Therapy 2:27-32 (1991); and Rettinger, et al., Proc. Natl. Acad. Sci. USA 91:1460-1464 (1994)).

### Ex Vivo Delivery of a Glucokinase Gene

### (a) In Vitro Transduction of Cells in Culture

Primary cells and cell lines are grown in the recommended media supplemented with the appropriate growth factors, serum, and antibiotics. The cells are transduced with the glucokinase gene either by direct infection with a recombinant viral vector described above or by non-viral delivery means, e.g., DNA-mediated transfection. Recombinant DNA expression cassettes comprising of cellular promoters/enhancers and regulatory regions operably linked to the glucokinase genes/cDNAs designed for expression in target mammalian tissues in the form of plasmids, linearized DNA fragments, or viral DNA/RNA vectors are prepared and purified as described in Sambrook, et al., Molecular Cloning - A Laboratory Manual (Cold Spring Harbor Laboratory, 1989). DNA can be introduced into cells by DNA-mediated transduction following one of the following methods: calcium phosphate precipitation, DEAE-Dextran method, electroporation (Ausudel, et al., Current Protocols in Molecular Biology (Wiley-Interscience, 1987)), lipofectin or protoplast fusion (Sandra-Goldin, et al., Mol. Cell Biol. 1:743-752 (1981)). Where the selectable marker is on a separate plasmid, the calcium phosphate co-precipitation method (Ausudel, et al., Current Protocols in Molecular Biology (Wiley-Interscience, 1987)) is used.

Twenty-four hours after DNA-mediated transfection, the cells in culture are trypsinized and replated in selection media at a density of 1/10. Clonal cell line that have inherited the selectable marker are picked by ring cloning, expanded in culture, and analyzed for the inheritance of the transfected gene of interest by PCR (Innis, et al., PCR Protocols: A Guide to Methods and Applications (Academic Press, 1990)) and Southern blot analysis (Southern, J. Mol. Biol. 98:503 (1975)) of genomic DNA prepared from the clonal cells/cell lines. Expression of the transfected glucokinase gene are examined by Northern blot analysis (Sambrook, et al., Molecular Cloning - A Laboratory Manual, (Cold Spring Harbor Laboratory, 1989) of total RNA by immunoblot analysis with glucokinase-specific antisera and by glucokinase activity assay (Valera, et al., Eur. J. Biochem. 222:533-539 (1994); Davidson, et al, Arch. Biochem. Biophys. 253:156-167 (1987)).

### (b) Ex Vivo Modification of Cells for Implantation

The technologies for virus and DNA-mediated gene transduction into mammalian cells (e.g., primary cells and cell lines) allow for *ex vivo* cellular engineering. These engineered cells can serve as metabolic factories (Newgard, Diabetes 43:341-350 (1994); Hughes, et al., Proc. Natl. Acad. Sci. USA 89:688-692 (1992); and Newgard, C.B., J. Lab. Clin. Med. 122:356-363 (1993)), as novel drug delivery systems (Kasid, et al., Proc. Natl. Acad. Sci. USA 87:473-477 (1990); and Chen, et al., Human Gene Therapy 6:917-926 (1995)), as surrogate tissues or organs (Mendell, J.R., et al., N. Engl. J. Med. 333:832-838 (1995); and Rhim, et al., Science 263:1149-1152 (1994)), or as neo-organs (Thompson, et al., Proc. Natl. Acad. Sci. USA 86:7928-7932 (1989)); and Culliton, Science 246:747-749 (1989)) upon appropriate implantation into the patient.

For example, hepatocytes can be isolated from the liver (Ponder, et al., Proc. Natl. Acad. Sci. USA 1217-1221 (1991); and Pages, et al., Human Gene Therapy 6:21-30 (1995)), committed to short term culture (Pages, et al., Human Gene Therapy 6:21-30 (1995)), and then transduced with a viral or plasmid vector carrying the expression cassette comprising of the glucokinase cDNA under the transcriptional control of a liver specific promoter. The genetically modified hepatocytes are then harvested and transplanted into a patient either by infusion of the cells into the portal vein (Wilson, et al., Proc. Natl. Acad. Sci. USA 87:6437-8441 (1990)) or by intrasplenical introduction (Ponder, et al., Proc. Natl. Acad. Sci. USA 88:1217-1221 (1991)). In mice, genetically modified hepatocytes introduced intrasplenically were shown to replace up to 80% of the diseased liver (Rhim, et al., Science 263:1149-1152 (1994)). In dogs, a 5% replacement of the liver mass with hepatocytes transduced with the human "1-antitrypsin expressing retrovirus resulted in the expression of the human peptide for up to 30 days (Kay, et al., Proc. Natl. Acad. Sci. USA 89:89-93 (1992)). Similarly, hypercholesterolemia in Watanabe heritable hyperlipidemic rabbits were transiently corrected by implantation of hepatocytes transduced with a retrovirus capable of directing the expression of a functional low density lipoprotein ("LDL") receptor (Wilson, Proc. Natl. Acad. Sci. USA 87:8437-8441 (1990)). An internal liver specific promoter could enhance sustained level of expression of the transgene.

In another example, myoblasts can be isolated from muscle biopsies (Mendell, et al., N. Engl. J. Med. 832-838 (1995)), expanded in culture, and genetically modified to express high, levels of glucokinase by transfection with DNA comprising of the glucokinase gene under the transcription control of a strong muscle specific promoter/enhancer or infected with a muscle specific recombinant retrovirus (Ferrari, et al., Human Gene Therapy 6:733-742 (1995)). The glucokinase expressing myoblasts can then be transferred into muscle by direct injection of the cells. Previous experience with murine myoblast have demonstrated that the injected myoblasts will fuse into pre-existing multinucleate myofibrils (Dhawan, et al., Science 254:1509-1512 (1991); and Barr, et al., Science 254:1507-1509 (1991)). The differentiated muscle fibers will maintain a high level of expression of the transgene (Yao, et al., Proc. Natl. Acad. Sci. USA 89:3357-3361 (1992)).

In still another example, human fibroblasts can be modified by receptor mediated or retroviral mediated gene transfer (Veelken, et al., Human Gene Therapy 5:1203-1210 (1994); and Chen, et al., Human Gene Therapy 6:917-926 (1995)) to overexpress glucokinase. The cells are then embedded into collagen coated lattices of expanded polytetrafluoroethylene (Gore-Tex) fibers as previously described (Thompson, et al., Proc. Natl. Acad. Sci. USA 86:7928-7932 (1989); and Moullier, et al., Nature Genetics 4:154-159 (1993)). Adsorption of heparin-binding growth factors-1 to the collagen lattices, upon implantation into the peritoneal cavity, will induce vascularization and formation of a neo-organ. Such neo-organs were reported to produce a sustained expression of transgenes in mice (Salvetti, et al., Human Gene Therapy 6:1153-1159 (1995)) and dogs (Moullier, et al., Nature Med. 1:353-357 (1995)). Such neo-organs comprising of fibroblast or other types of cells overexpressing glucokinase can serve to normalize blood sugars in the insulinopenic state.

### In Vivo Non-Viral Delivery of a Glucokinase Gene into Patients

The non-viral glucokinase gene constructs can also be targeted *in vivo* to specific tissue or organs, e.g., the liver or muscle, in patients. Examples of such delivery systems include receptor mediated endocytosis, liposome encapsulation, or direct insertion of non-viral expression vectors.

### (a) Receptor Mediated Endocytosis

By receptor mediated endocytosis, the glucokinase gene can be delivered to specific cells, e.g., hepatocytes in the liver (Wu, et al., J. Biol. Chem. 266:14338-14342 (1991); and Wilson, et al., J. Biol. Chem. 267:963-967 (1992)). Using asialoorosomucoid coupled to poly-L-lysine, soluble glucokinase DNA complexed to this molecule can be selectively delivered to hepatocytes in the liver via binding to the asialoglycoprotein receptor followed by endocytosis. The ligand-DNA complex is administered intravenously. A transient alleviation of hypercholesterolemia has been obtained with this technology by delivering functional LDL receptors into LDL receptor deficient rabbits (Wilson, et al., J. Biol. Chem. 267:963-967 (1992)). Other DNA-ligand conjugates have been developed, e.g., transferrin-polylysine-DNA complexes (Wagner, et al., Proc. Natl. Acad. Sci. USA 87:3410-3414 (1990); Wagner, et al., Proc. Natl. Acad. Sci. USA 89:7934-7938 (1992); and Wagner, et al., Proc. Natl. Acad. Sci. USA 89:6099-6103 (1992)), surfactant B-polylysine-DNA complexes (Baatz, et al., Proc. Natl. Acad. Sci. USA 91:2547-2551 (1994)), and anti-thrombomodulin-polylysine-DNA complexes (Trubetskoy, et al., Bioconjug. Chem. 3:323-327 (1992)).

### (b) Liposome Encapsulation

Successful *in vivo* gene transfer has been achieved with the injection of DNA, e.g., as a linear construct or a circular plasmid, encapsulated in liposomes (Ledley, Human Gene Therapy 6:1129-1144 (1995) and Farhood, et al., Ann. NY Acad. Sci. 716:23-35 (1994)). A number of cationic liposomes amphiphiles are now in development (Ledley, Human Gene Therapy 6:1129-1144 (1995); Farhood, et al., Ann. NY Acad. Sci., 716:23-35 (1994).

Intratracheal administration of cationic lipid-DNA complexes was shown to effect gene transfer and expression in the epithelial cells lining the bronchus (Brigham, et al., Am. J. Respir. Cell Mol. Biol. 8:209-213 (1993); and Canonico, et al., Am. J. Respir. Cell Mol. Biol. 10:24-29 (1994)). Expression in pulmonary tissues and the endothelium was reported after intravenous injection of the complexes (Brigham, et al., Am. J. Respir. Cell Mol. Biol. 8:209-213 (1993); Zhu, et al., Science, 261:209-211 (1993); Stewart, et al., Human Gene Therapy 3:267-275 (1992); Nabel, et al., Human Gene Therapy 3:649-656 (1992); and Canonico, et al., J. Appl. Physiol. 77:415-419 (1994)). The expression cassettes for glucokinase in linear, plasmid or viral DNA forms can be condensed through ionic interactions with the cationic lipid to form a particulate complex for *in vivo* delivery (Stewart, et al., Human Gene Therapy 3:267-275 (1992)).

Other liposome formulations, for example, proteoliposomes which contain viral envelope receptors proteins, i.e., virosomes, have been found to effectively deliver genes into hepatocytes and kidney cells after direct injection (Nicolau, et al., Proc. Natl. Acad. Sci. USA 80:1068-1072 (1993); Kaneda, et al., Science 243:375-378 (1989); Mannino, et al., Biotechniques 6:682 (1988); and Tomita, et al., Biochem. Biophys. Res. Comm. 186:129-134 (1992)).

### (c) Direct Injection

Direct injection of glucokinase DNA expression vectors, e.g., into the muscle or liver, either as a solution or as a calcium phosphate precipitate (Wolff, et al., Science 247:1465-1468 (1990); Ascadi, et al., The New Biologist 3:71-81 (1991); and Benvenisty, et al., Proc. Natl. Acad. Sci. USA 83:9551-9555 (1986)), provides alternative technology for delivering the glucokinase expression cassettes into tissues of recipients.

Microinjection of the glucokinase DNA segment, e.g., as a linear construct or a circular plasmid, into the pronucleus of a zygote or two-cell embryos that could transmit the transgene to subsequent generations constitutes an approach to achieving germ line gene therapy (Hogan, et al., Manipulating the Mouse Embryo, Cold Spring Harbor Laboratory, 1986); Brinster, et al., Proc. Natl. Acad. Sci. USA 82:4438-4442 (1985)). Another method for achieving germ-line gene therapy is effecting gene transfection and homologous recombination in embryonic stem cells (Thomas, et al., Cell 51:503-512 (1987); and Capecchi, Science 244:1288-1292 (1989)).

The following are examples which demonstrates certain aspects of how to practice the therapeutic process of this invention. It is believed that one skilled in the art, based upon the description herein, can utilize the invention to its fullest extent. The specific examples set forth below are, therefore to be construed as merely illustrative and not limitative of the remainder of the disclosure whatsoever.

### Example 1: Expression of Glucokinase in the Liver

### (a) Construction of the Rat PEPCK Promoter/Rat Glucokinase (PEPCK/GK) Chimeric Gene

All DNA modifying enzymes were purchased from Boehringer-Mannheim, Germany. To obtain the PEPCK/GK chimeric gene, a 2.3 kb EcoRI-EcoRI fragment containing the entire coding sequence and the polyadenylation signal of the rat glucokinase cDNA was used (Iynedjian, et al., J. Biol. Chem. 262:6032-6038 (1987)). The cDNA was introduced at an EcoRI site newly created in the PEPCK/insulin chimeric gene (Valera, A., et al., FASEB 8:440-447 (1994)). This chimeric gene was used as a liver expression vector to obtain regulated expression and mRNA stabilization from cDNAs in transgenic mice. The strategy used to obtain the PEPCK/GK chimeric gene was initiated by subcloning the BglII-SphI fragment of the PEPCK/insulin chimeric gene, which includes the first exon (3'-Ins) and the translation initiation site of the human insulin gene (5'-Ins), at the pSP72 polylinker. To destroy the ATG translation start site, which was contained in an unique NcoI restriction site, this fragment of the insulin gene was digested with NcoI and treated with mung bean nuclease, to remove the single-stranded overhangs produced by the restriction enzyme. Afterwards, an EcoRI linker (Boehringer Mannheim, Germany) was introduced to generate a new EcoRI subcloning site. Then, the EcoRI-EcoRI fragment of glucokinase cDNA was introduced at this new EcoRI site. Finally, the BglII-SphI fragment of the human insulin gene containing the full-length glucokinase cDNA was reinserted into the PEPCK/insulin chimeric gene. The final plasmid was designated pPEPCK/GK.

### (b) Construction of Transgenic Mice

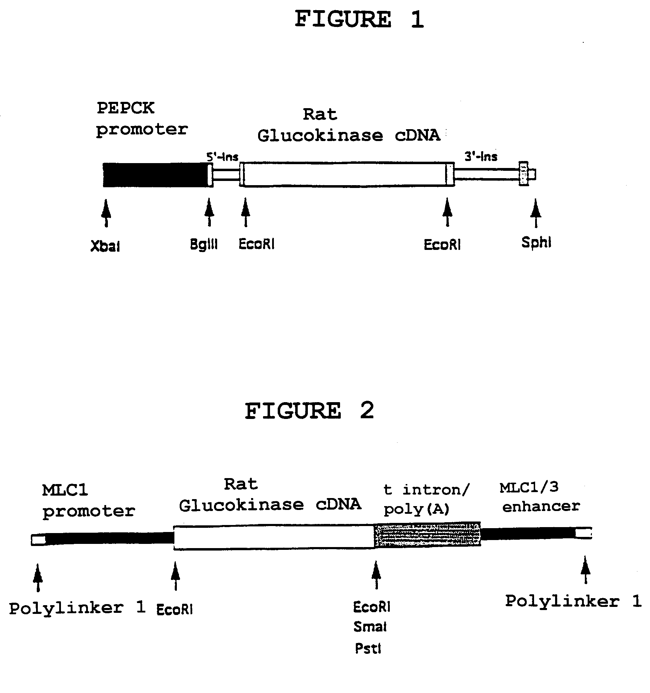

A 4.5 kb XbaI-SphI fragment, containing the entire PEPCK/GK chimeric gene, was microinjected into fertilized eggs flushed from the oviducts of superovulated C57BL6/HJL mice 6-8 hours after ovulation. A schematic diagram of the chimeric gene is depicted in Fig. 1. The expression of this chimeric gene lead to a 2.5 kb mRNA transcript when polyadenylated at the signal at the end of the glucokinase cDNA. The general procedures for microinjection of the chimeric gene were as described in Hogan, B., et al., Manipulating the Mouse Embryo - A Laboratory Manual, Cold Spring Harbor Laboratory Press (1986). At 3 weeks of age, the animals were tested for the presence of the transgene by Southern blot of 10 :g of tail DNA digested with EcoRI. Blots were hybridized with a 2.3 kb EcoRI-EcoRI fragment containing the entire glucokinase cDNA radiolabeled with ["-³²P]dCTP (3000 Ci/mmol), Amersham Corp., Arlington Heights, IL) by random oligopriming (Boehringer-Mannheim, Germany). Animals expressing the transgene were kept and were fed ad libitum with a standard diet (Panlab, Barcelona, Spain) and maintained under a light-dark cycle of 12 h (lights on at 8:00 a.m.). For the diabetic group, diabetes was induced by injection through the jugular vein of doses of 2 mg streptozotocin/10 g body weight on 2 consecutive days. Streptozotocin ("Stz") (Sigma Chemical Co., Saint Louis, MO) was dissolved in a 10 mM sodium citrate with 0.9% NaCl, pH 4.5, immediately before administration. Mice were used 7 days after streptozotocin treatment. Diabetes was assessed by measuring glycemic, glycosuric and ketonuric levels (ACCUTREND and GLUKETUR Test, Boehringer Mannheim, Germany). Animals were sacrificed, and samples were taken between 9-10 a.m. Male mice aged 4 to 8 weeks were used.

F1 and F2 generation mice from two transgenic lines were found to carry about 5 and 20 intact copies, respectively, of the PEPCK/GK chimeric gene as analyzed by Southern blot, and were used in further studies. Litter-mates were used as controls for the transgenic animals.

### (c) Expression of Chimeric Gene

Total RNA was obtained from liver by the guanidine isothiocyanate method as described in Chirgwin, et al., Biochemistry 18:5294-5299 (1979), and RNA samples (30 :g) were electrophoresed on a 1% agarose gel containing 2.2 M formaldehyde. Northern blots were hybridized to a ³²P-labeled glucokinase cDNA probe and a ³²P-labeled $-actin probe corresponding to a 1.3 kb EcoRI-EcoRI fragment of the rabbit cDNA as indicated in Valera, A., et al., FASEB J. 9:1067-1078 (1995). These probes were labeled with ["-³²P]dCTP, following the method of random oligopriming as described by the manufacturer. Specific activity of the labeled DNA probe was approximately 10⁹ cpm/:g DNA. Membranes were placed in contact with KODAK XAR-5 films. The $-actin signal was used to normalise signal intensities for comparison across lanes.

### (d) Preparation and Incubation of Hepatocytes

Hepatocytes were isolated, between 10-11 a.m., from fed normal and diabetic mice as described in LeCam, A., et al., Exp. Cell Res. 98:382-395 (1976). After removing non-parenchymal cells and debris, the hepatocytes were resuspended in Dulbecco's modified Eagle's medium ("DMEM") (Gibco, Grand Island, NY) containing 0.2% albumin and 10% fetal calf serum (Boehringer-Mannheim, Germany). 5.5 x 10⁶ cells were plated in 10 ml of this medium on collagen-coated dishes and maintained at 37°C under a 5% CO₂ atmosphere. After four hours, the medium was removed and cells were washed three times in DMEM in the absence of either serum or glucose. Subsequently, 10 ml of DMEM without serum and with 25 mM glucose was added to the cells, which were maintained in this medium for up to 24 h. Aliquots of 100:l of medium were taken at different times and lactate production was determined. To measure glucose and ketone body production, hepatocytes were incubated in 10 ml of DMEM without glucose and supplemented with 16 mM lactate plus 4 mM pyruvate for up to 24 h. Aliquots of 100 ml of medium were taken at different times and glucose and $-hydroxybutyrate concentrations were determined.

### (e) Enzyme, Metabolite, and Hormone Assays

To measure glucokinase activity, liver samples were homogenized in an ice-cold buffer (pH 7.4) containing 50 mM Tris-HCl, 300 mM sucrose, 100 mM KCl, 1 mM ethylenediamine tetraacetic acid ("EDTA"), and 0.7 :l/ml $-mercaptoethanol. To determine pyruvate kinase activity, the liver was homogenised in an ice-cold buffer containing 100 mM KCl, 1 mM EDTA, and 0.7:l/ml $-mercaptoethanol. These activities were analysed in 12,000 x g supernatants as described in Davidson, A.L., et al., Arch. Biochem. Biophys. 253:156-176 (1987) and Feliu, J.E., et al., Eur. J. Biochem. 81:609-617 (1977). lucokinase activity was calculated as the difference between the glucose phosphorylation capacity at 100 and 0.5 mM glucose, and hexokinase activity as the glucose phosphorylating capacity at 0.5 mM glucose. Pyruvate kinase activity was determined at 5 mM phosphoenolpyruvate (total activity). The concentrations of insulin and glucose in serum, glycogen, glucose 6-phosphate and lactate in liver extracts, glucose, and lactate in the incubation media of hepatocytes were measured as described in Valera, et al., FASEB J. 9:1067-1078 (1995). Glucose was also determined in 20 ml of blood by using an ACCUTREND analyzer (Boehringer-Mannheim, Germany). The $-hydroxybutyrate levels in serum and in the incubation medium of hepatocytes were measured by the $-hydroxybutyrate dehydrogenase technique (Boehringer-Mannheim, Germany). Serum triglycerides were determined enzymatically (GPO-PAP, Boehringer-Mannheim, Germany). Serum-free fatty acids were measured by the acyl-CoA synthase and acyl-CoA oxidase method (Wako Chemicals, Germany). Enzyme activities and metabolite concentrations are expressed as the means " SEM.

### (f) Determination of Glucokinase Expression

A 2.5 kb transcript was detected in the liver of control and transgenic mice, resulting from the expression of both the endogenous glucokinase gene and also the glucokinase transgene. No glucokinase mRNA transcripts were detected in the liver of diabetic control mice, while Stz-treated transgenic mice retained a high level of glucokinase mRNA expression. Glucokinase activity in the liver of healthy transgenic mice was 2-fold higher in both transgenic lines than in controls.

The enzyme activity in Stz-diabetic control mice was extremely low. The glucokinase activities of Stz-treated mice in both lines were similar to or higher than that noted in healthy control mice. These findings suggest that the gene product made from the glucokinase transgene was stable and active in a diabetic environment.

### (g) Glucose Storage and Utilization

The results reported below in Table I were obtained from the transgenic line which carried about 20 intact copies of the PEPCK/GK gene. In both Tables I and II, "Con" and "Tg" stand for control mice and transgenic mice, respectively.

**TABLE I**

| | NON-TREATED | | STZ-TREATED | |
|---|---|---|---|---|
| | Con | Tg | Con | Tg |
| GLUCOSE 6-PO₄ (nmol/g liver) | 215 ± 15 | 337 ± 21 | 56 ± 12 | 230 ± 26 |
| GLYCOGEN (mg/g liver) | 49 ± 6 | 98 ± 9 | 4 ± 2 | 44 ± 5 |
| PYRUVATE KINASE | 0.24 ± 0.02 | 0.53 ± 0.06 | 0.15 ± 0.03 | 0.59 ± 0.08 |
| LACTATE (:mol/g liver) | 0.23 ± 0.03 | 0.44 ± 0.04 | 0.06 ± 0.02 | 0.25 ± 0.4 |

As shown in Table I, the reduction of glucokinase activity led to a 75% decrease in the intracellular concentration of glucose-6-PO₄ in diabetic control mice compared with healthy controls. In contrast, the level of glucose-6-PO₄ in Stz-treated transgenic mice were similar to those of healthy control mice. Glucose-6-PO₄ is a substrate for the synthesis of glycogen as well as an allosteric activator of glycogen synthase (Larner, J., Adv. Enzymol. Relat. Areas Mol. Biol. 63:173-231 (1990)). Transgenic mice expressing the glucokinase transgene accumulated more glycogen (about 2-fold) than control mice. In diabetes, the lack of insulin and an increase in glucagon results in the phosphorylation and inactivation of glycogen synthase. No glycogen was stored in the liver of Stz-diabetic control mice. However, Stz-treated transgenic mice retained the capacity to synthesize glycogen at a level similar to that in control healthy mice. This is in accordance with the increase in intrahepatic glucose 6-PO₄. Thus, the expression of the glucokinase transgene alone was sufficient to restore normal glycogen synthesis in the insulinopenic state.

An induction of glucose utilization is evident in the increase pyruvate kinase activity and intrahepatic lactate concentration in the glucokinase transgenic animal compared to controls.

### (h) Serum Parameters

The changes in liver metabolism caused a normalisation of serum parameters altered during diabetes as reported in Table II. Note that "FFAs" stands for free fatty acids.

**TABLE II**

| | NON-TREATED | | STZ-TREATED | |
|---|---|---|---|---|
| | Con | Tg | Con | Tg |
| GLUCOSE (mg/dL) | 207 ± 11 | 148 ± 9 | > 800 | 265 ± 14 |
| INSULIN (ng/mL) | 2.7 ± 0.3 | 1.5 ± 0.1 | < 0.2 | < 0.2 |
| $-OH-butyrate (mmol/L) | 0.37 ± 0.06 | 0.30 ± 0.07 | 2.94 ± 0.3 | 0.49 ± 0.12 |
| TRIGLYCERIDES (mg/dL) | 129 ± 15 | 154 ± 12 | 365 ± 28 | 181 ± 15 |
| FFAs (mmol/L) | 0.98 ± 0.1 | 0.78 ± 0.1 | 1.95 ± 0.2 | 0.83 ± 0.1 |

As shown in Table II, Stz-control mice exhibited insulinopenia and hyperglycemia, characteristic of diabetes mellitus. Stz-treated transgenic mice on the other hand showed plasma glucose concentrations close to the normal range, despite a very low level of insulin. These results suggest a major role of the liver in whole body glucose homeostasis and a key role for glucokinase in the regulation of hepatic glucose uptake and metabolism. An induction of ketogenesis is a common feature of untreated insulin-dependent diabetes mellitus (Taylor, et al., Biochem. J. 250:625-640 (1988); and McGarry, J.D., Science 258:766-770 (1992)). The high concentration of the ketone body $-hydroxybutyrate ($-OH-butyrate) in the serum of Stz-treated control mice is evidence of ketosis. In contrast, ketone body production remains within the normal range in the Stz-treated transgenic mice. The expression of the glucokinase transgene also led to a normalization of circulating triglycerides and free fatty acids, which are markedly increased during diabetes. These results indicated that the rescue of glucose uptake by the liver was sufficient to normalize glucose, lipid and ketone body sufficient to normalize glucose, lipid and ketone body metabolism. Consistent with the diabetic state, Stz-treated control mice exhibited a 25% reduction in body weight fifteen days after treatment with streptozotocin (from 21.4 " 0.5 g to 15.7 " 0.8 g (n=25)). The normalization of liver metabolism and serum parameters in the Stz-treated transgenic mice was accompanied by the maintenance of body weight (from 20.3 " 0.6 g to 21.1 " 0.9 g (n=25)).

In summary, the data suggests that the delivery and expression of glucokinase to a tissue like the liver is sufficient to provide protection of the organism from developing diabetes mellitus despite insulinopenia.

### Example 2: Expression of Glucokinase in the Muscle

### (a) Construction of the Rat Myosin Light Chain-1/Rat Glucokinase (MLC1/GK) Chimeric Gene

To obtain the MLC1/GK chimeric gene, a 2.3 kb *Eco*RI-*Eco*RI fragment containing the entire coding sequence and the polyadenylation signal of the rat glucokinase cDNA was used. A 1.5 kb *Eco*RI/HindII fragment of the MLC1 promoter, including the cap site and spanning 105 bp of untranslated sequences, was fused to the 5' end of the glucokinase cDNA (Periasamy, et al., J. Biol. Chem. 259:13595-13604 (1984). To increase stabilization of the mRNA of the transgene, a 0.8 kb fragment containing the small intron and the polyadenylation signal of SV40 t antigen (t intron/poly (A)) was introduced at the 3' end of the glucokinase cDNA (Laimins, et al., Proc. Natl. Acad. Sci. 79:6453-6457 (1982). Lastly, a 0.9 kb SphI/HindIII genomic fragment of the MLC1/3 gene, containing a strong muscle-specific enhancer (Donoghue, et al., Genes & Dev. 2:1779-1790 (1988) was introduced in the transgene at the 3' end of the SV40 fragment to ensure high levels of expression in skeletal muscle. The resulting plasmid was designated pMLC1/GK.

### (b) Construction of Transgenic Mice

A 5.5 kb XhoI-NotI fragment, containing the entire PEPCK/GK chimeric gene, was microinjected into fertilized eggs flushed from the oviducts of superovulated C57BL6/HJL mice 6-8 hours after ovulation. A schematic diagram of the chimeric gene is depicted in Fig. 2. The expression of this chimeric gene lead to a 2.5 kb mRNA transcript when polyadenylated at the signal at the end of the glucokinase cDNA. The general procedures for microinjection of the chimeric gene were as described in Hogan, et al., Manipulating the Mouse Embryo - A Laboratory Manual (Cold Spring Harbor Laboratory Press, 1986). At 3 weeks of age, the animals were tested for the presence of the transgene by Southern blot of 10 :g of tail DNA digested with *Eco*RI. Blots were hybridized with a 2.3 kb *Eco*RI-*Eco*RI fragment containing the entire glucokinase cDNA radiolabeled with ["-³²P]dCTP (3000 Ci/mmol, Amersham Corp., Arlington Heights, IL) by random oligopriming. Mice were fed *ad libitum* with a standard diet (Panlab, Barcelona, Spain) and maintained under a light-dark cycle of 12 h (lights on at 8:00 a.m.). Male mice aged 4 to 8 weeks were used.

### (c) Expression of Chimeric Gene

Total RNA was obtained from skeletal muscle by the guanidine isothiocyanate method (Chirgwin, et al., Biochemistry 18:5294-5299 (1979)), and RNA samples (30 :g) were electrophoresed on a 1% agarose gel containing 2.2 M formaldehyde. Northern blots were hybridized to ³²P-labeled glucokinase cDNA probe as indicated in (Valera,, et al., FASEB J. 9:1067-1078 (1995). This probe was labeled using ["-³²P]dCTP, following the method of random oligopriming as described by the manufacturer (Boehringer-Mannheim, Germany). Specific activity of the labeled DNA was approximately 10⁹ cpm/:g DNA. Membranes were placed in contact with Kodak XAR-5 films.

### (d) Enzyme and Metabolite Assays

To measure glucokinase and hexokinase activity, skeletal muscle samples were homogenized in an ice-cold buffer (pH 7.4) containing 50 mM Tris-HCl, 300 mM sucrose, 100 mM KCl, 1 mM ethylenediamine tetraacetic acid (EDTA), and 0.7:l/m $-mercaptoethanol. These activities were analysed in 12,000 x g supernatants (Davidson, et al., Arch. Biochem. Biophys. 253:156-176 (1987) and Feliu, et al., Eur. J. Biochem. 81:609-617 (1977). Glucokinase activity was calculated as the difference between the glucose phosphorylation capacity at 100 and 5 mM glucose and hexokinase activity as the glucose phosphorylating capacity at 5 mM glucose. The concentrations of insulin and glucose in serum, glycogen, glucose 6-PO₄, and lactate in muscle extracts were measured as described in Valera,, A., et al., FASEB J. 9:1067-1078 (1995). Glucose was also determined in 20 ml of blood by using an Accutrend® analyser (Boehringer-Mannheim).

In this study, F1 and F2 mice from the transgenic line MLC1/GK-A were used which carried about 10 intact copies of the chimeric gene when analyzed by Southern blot. Littermates were used as controls for the transgenic animals. A 2.5 kb transcript was detected in the skeletal muscle of transgenic mice, resulting from the expression of the transgene. No glucokinase mRNA transcripts were detected in the muscle of control mice. The increase in the expression of glucokinase in the muscle of transgenic mice was parallel to the activation of the enzyme. These findings suggest that glucokinase mRNA was translated and that the protein was stable in an ectopic environment, such as muscular tissue. These results also indicated that the establishment of some level of glucokinase mRNA in the muscle of transgenic mice was enough to produce highly activated enzyme.

An intraperitoneal glucose tolerance test performed on these transgenic mice, either from line MLC1/GK-A or with other lines of MLC1/GK, showed a marked reduction (about 35%) in the blood glucose levels as compared to control. The increase in circulating glucose levels was transient, and gradually returned to basal levels by 120 min in the transgenic mice and by 180 min in the controls.

### Other Embodiments

It is to be understood that while the invention has been described in conjunction with the detailed description thereof, that the foregoing description is intended to illustrate and not limit the scope of the invention, which is defined by the scope of the appended claims. Other aspects, advantages, and modifications are within the claims.

## Claims

1. Use of a DNA segment which includes a glucokinase gene and a promoter sequence, said promoter sequence being operably linked to said glucokinase gene and being effective for the expression of a therapeutically effective amount of glucokinase in a patient, for the manufacture of a medicament for the treatment of diabetes.

2. The use of claim 1, wherein said DNA segment is introduced to the patient in cells, said cells having been treated *in vitro* to incorporate therein said DNA segment.

3. The use of claim 2, wherein said cells express a glucose transporter.

4. The use of claim 3, wherein said glucose transporter is GLUT-2 and GLUT-4.

5. The use of any one of claims 2 to 4, wherein said cells are liver cells or muscle cells.s

6. The use of any one of claims 1 to 5 wherein said promoter sequence is different from the promoter sequence controlling transcription of endogenous glucokinase.

7. The use of any one of claims 1 to 6, wherein said promoter sequence is inducible during diabetic conditions.

8. The use of any of claims 1 to 7, wherein said promoter is a PEPCK promoter or a MLC1 promoter.

9. The use of any one of claims 2 to 8, wherein said DNA segment has been incorporated in said cells by a viral vector.

10. The use of claim 9, wherein said viral vector is a retroviral vector.

11. The use of claims 2 to 10, wherein cells are in a neo-organ.

12. The use of claim 1, wherein said DNA segment is administered directly to a patient.

13. The use of claim 12, wherein said DNA segment is an integral part of a vector.

14. The use of claim 13, wherein said vector is a viral vector.

15. The use of claim 14, wherein said vector is a retroviral vector, an adenoviral vector, and adeno-associated vector, a sindbis viral vector, or a herpes viral vector.

16. The use of claim 12, wherein said DNA segment is administered to said patient in a DNA-ligand conjugate, a liposome, or a calcium phosphate precipitate.

17. A process of treating a diabetic patient, which comprises the step of administering to said patient a DNA segment which includes a glucokinase gene and a promoter sequence, said promoter sequence being operably linked to said glucokinase gene and being effective for the expression of a therapeutically effective amount of glucokinase in said patient.

18. A process of claim 17, wherein said DNA segment is introduced to said patient in cells, said cells having been treated *in vitro* to incorporate therein said DNA segmemt.

19. A process of claim 18, wherein said cells express a glucose transporter.

20. A process of claim 19, wherein said glucose transporter is GLUT-2 or GLUT-4.

21. A process of claims 18 to 20, wherein said cells are liver cells or muscle cells.

22. A process of any one of claims 17 to 21, wherein said promoter sequence is different from the promoter sequence controlling transcription of endogenous glucokinase.

23. A process of any one of claims 17 to 22, wherein said promoter sequence is inducible during diabetic conditions.

24. A process of any of claims 17 to 23, wherein said promoter is a PEPCK promoter or a MLC1 promoter.

25. A process of any one of claims 18 to 24, wherein said DNA segment has been incorporated in said cells by a viral vector.

26. A process of claim 25, wherein said viral vector is a retroviral vector.

27. A process of any one of claims 18 to 26, wherein said cells are in a neo-organ.

28. A process of claim 17 wherein said DNA segment is administered directly to said patient.

29. A process of claim 28, wherein said DNA segment is an integral part of a vector.

30. A process of claim 29, wherein said vector is a viral vector.

31. A process of claim 30, wherein said vector is a retroviral vector, an adenoviral vector, and adeno-associated vector, a sindbis viral vector, or a herpes viral vector.

32. A process of claim 28, wherein said DNA segment is administered to said patient in a DNA-ligand conjugate, a liposome, or a calcium phosphate precipitate.

33. A recombinant DNA molecule comprising a glucokinase gene and a promoter sequence, in which the promoter sequence is operably linked to the glucokinase gene.

34. The DNA molecule of claim 33 wherein the promoter sequence is different from the promoter sequence controlling transcription of endogenous glucokinase.

35. The DNA molecular of claim 34 wherein the promoter is PEPCK promoter or MLC1 promoter.

36. An expression vector comprising a DNA molecule as defined in any one of claims 33 to 35.

37. The expression vector of claim 36 wherein the vector is a viral vector.

38. A pharmaceutical composition comprising a DNA molecule as defined in any one of claims 33 to 35.

39. A cell comprising a DNA molecule as defined in any one of claims 33 to 35.

40. A DNA molecule or expression vector as defined in any one of claims 33 to 37 for use in therapy.

41. A DNA molecule or expression vector as defined in any one of claims 33 to 37 for use in the treatment of diabetes.
